# EUROPÄISCHE PATENTSCHRIFT

(11) **EP 1 423 088 B1**
(45) Veröffentlichungstag und Bekanntmachung des Hinweises auf die Patenterteilung: **26.03.2008**
(21) Anmeldenummer: 02779270.4
(22) Anmeldetag: 22.08.2002
(51) Int. Cl.: A61K 8/37, A61K 8/67, A61K 8/60, A61K 8/49, A61Q 19/00, A61Q 19/08, A61Q 17/04

(54) **STABILISIERUNG OXIDATIONS- UND/ODER UV-EMPFINDLICHER WIRKSTOFFE**
STABILISATION OF OXIDATION-SENSITIVE AND UV-SENSITIVE ACTIVE INGREDIENTS
STABILISATION DE PRINCIPES ACTIFS SENSIBLES A L'OXYDATION ET/OU AUX U.V.

(30) Priorität: 29.08.2001 DE 10141472
(43) Veröffentlichungstag der Anmeldung: 02.06.2004
(73) Patentinhaber: Beiersdorf AG, 20245 Hamburg (DE)
(72) Erfinder: WENDEL, Volker, 20255 Hamburg (DE); GÖPPEL, Anja, 22527 Hamburg (DE)
(86) Internationale Anmeldenummer: PCT/EP2002/009374
(87) Internationale Veröffentlichungsnummer: WO 2003/020235

(56) Entgegenhaltungen:
- EP-A- 1 129 696
- WO-A-01/89465
- FR-A- 2 801 208
- FR-A- 2 801 209
- US-A- 6 126 925

## Beschreibung

Die vorliegende Erfindung betrifft die Verwendung von Alkylnaphthalaten zur Verbesserung der Wirksamkeit von hydrophilen Wirkstoffen in kosmetischen oder dermatologischen Zubereitungen. Bevorzugt betrifft die vorliegende Erfindung die Verwendung von Alkylnaphthalaten zur Verbesserung der Wirksamkeit von wasserlöslichen Hautpflegestoffen - wie beispielsweise Biotin, Carnitin, Flavonoiden wie alpha-Glucosylrutin, Ascorbinsäure und dergleichen - und kosmetische oder dermatologische Zubereitungen, in welchen derartige Wirkstoffe über einen langen Zeitraum stabil gelagert werden können und welche ein gutes Transportmittel für diese Wirkstoffe darstellen.

Kosmetische Hautpflege dient in erster Linie dazu, die natürliche Funktion der Haut als Barriere gegen Umwelteinflüsse (z. B. Schmutz, Chemikalien, Mikroorganismen) und gegen den Verlust von körpereigenen Stoffen (neben Wasser auch natürliche Fette, Elektrolyte etc.) zu stärken oder wiederherzustellen.

Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliches Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Die chronologische Hautalterung wird z. B. durch endogene, genetisch determinierte Faktoren verursacht. In Epidermis und Dermis kommt es alterungsbedingt z. B. zu folgenden Strukturschäden und Funktionsstörungen, die auch unter den Begriff "Senile Xerosis" fallen können:
a) Trockenheit, Rauhigkeit und Ausbildung von Trockenheitsfältchen,
b) Juckreiz und
c) verminderte Rückfettung durch Talgdrüsen (z. B. nach Waschen).

Exogene Faktoren, wie UV-Licht und chemische Noxen, können kumulativ wirksam sein und z. B. die endogenen Alterungsprozesse beschleunigen bzw. sie ergänzen. In Epidermis und Dermis kommt es insbesondere durch exogene Faktoren z. B. zu folgenden Strukturschäden- und Funktionsstörungen in der Haut, die über Maß und Qualität der Schäden bei chronologischer Alterung hinausgehen:
d) Sichtbare Gefäßerweiterungen (Teleangiektasien, Cuperosis);
e) Schlaffheit und Ausbildung von Falten;
f) lokale Hyper-, Hypo- und Fehlpigmentierungen (z. B. Altersflecken) und
g) vergrößerte Anfälligkeit gegenüber mechanischem Stress (z. B. Rissigkeit).

Die vorliegende Erfindung betrifft insbesondere Produkte zur Pflege der auf natürliche Weise gealterten Haut sowie zur Behandlung der Folgeschäden der Lichtalterung, insbesondere der unter a) bis g) aufgeführten Phänomene, vorzugsweise solche, welche sich durch eine gesteigerte Wirksamkeit auszeichnen.

Insbesondere betrifft die vorliegende Erfindung kosmetische Zubereitungen mit einem wirksamen Schutz vor schädlichen Oxidationsprozessen in der Haut, aber auch zum Schutze kosmetischer Zubereitungen selbst bzw. zum Schutze der Bestandteile kosmetischer Zubereitungen vor schädlichen Oxidationsprozessen.

Die vorliegende Erfindung betrifft ferner Antioxidantien, bevorzugt solche, welche in hautpflegenden kosmetischen oder dermatologischen Zubereitungen eingesetzt werden. Insbesondere betrifft die Erfindung auch kosmetische und dermatologische Zubereitungen, solche Antioxidantien enthaltend. In einer bevorzugten Ausführungsform betrifft die vorliegende Erfindung kosmetische und dermatologische Zubereitungen zur Prophylaxe und Behandlung kosmetischer oder dermatologischer Hautveränderungen wie z. B. der Hautalterung, insbesondere der durch oxidative Prozesse hervorgerufenen Hautalterung.

Weiterhin betrifft die vorliegende Erfindung Wirkstoffe und Zubereitungen, solche Wirkstoffe enthaltend, zur kosmetischen und dermatologischen Behandlung oder Prophylaxe erythematöser, entzündlicher, allergischer oder autoimmunreaktiver Erscheinungen, insbesondere Dermatosen.

Die vorliegende Erfindung betrifft in einer weiteren vorteilhaften Ausführungsform Wirkstoffkombinationen und Zubereitungen, die zur Prophylaxe und Behandlung der lichtempfindlichen Haut, insbesondere von Photodermatosen, dienen.

Die schädigende Wirkung des ultravioletten Teils der Sonnenstrahlung auf die Haut ist allgemein bekannt. In Abhängigkeit von ihrer jeweiligen Wellenlänge haben die Strahlen verschiedene Wirkungen auf das Organ Haut: Die sogenannte UV-C-Strahlung mit einer Wellenlänge, die kleiner als 290 nm ist, wird von der Ozonschicht in der Erdatmosphäre absorbiert und hat daher keine physiologische Bedeutung. Dagegen verursachen Strahlen im Bereich zwischen 290 nm und 320 nm, dem sogenannten UV-B-Bereich, ein Erythem, einen einfachen Sonnenbrand oder sogar mehr oder weniger starke Verbrennungen. Als ein Maximum der E-rythemwirksamkeit des Sonnenlichtes wird der engere Bereich um 308 nm angegeben.

Zum Schutz gegen UV-B-Strahlung sind zahlreiche Verbindungen bekannt, bei denen es sich beispielsweise um Derivate des 3-Benzylidencamphers, der 4-Aminobenzoesäure, der Zimtsäure, der Salicylsäure, des Benzophenons sowie des Triazins handelt.

Man hat lange Zeit fälschlicherweise angenommen, daß die langwellige UV-A-Strahlung mit einer Wellenlänge zwischen 320 nm und 400 nm nur eine vernachlässigbare biologische Wirkung aufweist. Inzwischen ist allerdings durch zahlreiche Studien belegt, daß UV-A-Strahlung im Hinblick auf die Auslösung photodynamischer, speziell phototoxischer Reaktionen und chronischer Veränderungen der Haut weitaus gefährlicher als UV-B-Strahlung ist. Auch kann der schädigende Einfluß der UV-B-Strahlung durch UV-A-Strahlung noch verstärkt werden.

So ist es u. a. erwiesen, daß selbst die UV-A-Strahlung unter ganz normalen Alltagsbedingungen ausreicht, um innerhalb kurzer Zeit die Collagen- und Elastinfasern zu schädigen, die für die Struktur und Festigkeit der Haut von wesentlicher Bedeutung sind. Hierdurch kommt es zu chronischen lichtbedingten Hautveränderungen - die Haut "altert" vorzeitig. Zum klinischen Erscheinungsbild der durch Licht gealterten Haut gehören beispielsweise Falten und Fältchen sowie ein unregelmäßiges, zerfurchtes Relief. Ferner können die von lichtbedingter Hautalterung betroffenen Partien eine unregelmäßige Pigmentierung aufweisen. Auch die Bildung von braunen Flecken, Keratosen und sogar Karzinomen bzw. malignen Melanomen ist möglich. Eine durch die alltägliche UV-Belastung vorzeitig gealterte Haut zeichnet sich außerdem durch eine geringere Aktivität der Langerhanszellen und eine leichte, chronische Entzündung aus.

Es ist daher von grundsätzlicher Wichtigkeit, daß kosmetische und dermatologische Lichtschutzzubereitungen sowohl gegen UV-B- ais auch gegen UV-A-Strahlung ausreichenden Schutz bieten.

Die Schrift FR 2801208 offenbart kosmetische Bräunungszubereitungen mit einem Bräunungswirkstoff und bestimmten Derivaten der Naphthalincarbonsäuren.

Die Schrift FR 2801209 offenbart kosmetische Lichtschutzzubereitungen mit einem UV-Filtersystem, einer Öl- und einer Wasserphase und bestimmten Derivaten der Naphthalincarbonsäuren.

Die Schrift WO 0189465 offenbart AT- oder Deo-Zubereitungen in Form von Emulsionen, die unter anderem organische Naphthalatester mit bestimmten Brechungsindizes und bestimmten Löslichkeiten enthalten.

Die Schrift EP 1129696 offenbart synergistische Stoffkombinationen von 2-Ethylhexyl-2-cyano-3,3-diphenylacrylat mit Dialkylnaphthalaten.

Der Stand der Technik kennt ferner eine Reihe von verschiedenen effizienten, hydrophilen Hautpflegewirkstoffen - wie beispielsweise Biotin, Carnitin, Flavonoide wie alpha-Glucosylrutin, Ascorbinsäure und dergeichen -, deren Einsatz in kosmetischen und dermatologischen Formulierungen, insbesondere in Formulierungen vom Typ Öl-in-Wasser, sehr wünschenswert ist. Unglücklicherweise sind derartige Substanzen aber häufig sehr instabil, so daß sie insbesondere in wäßrigen Medien schnell zerfallen und auf diese Weise ihre Wirksamkeit verlieren. Da sie darüberhinaus schlecht öllöslich sind, läßt sich auch in nicht-wäßrigen Systemen nur schwer eine wirksame Konzentration an hydrophilen Wirkstoffen erreichen.

Weitere Aufgabe der vorliegenden Erfindung war es daher insbesondere, die Nachteile des Standes der Technik zu beseitigen und hydrophile Hautpflegewirkstoffe, insbesondere Biotin, Carnitin, Flavonoide wie alpha-Glucosylrutin und Ascorbinsäure, vor dem Zerfall zu schützen und so ihre Wirksamkeit zu erhöhen, und zwar bevorzugt in kosmetischen oder dermatologischen Zubereitungen. Ferner war Aufgabe, kosmetische oder dermatologische Zubereitungen zu finden, in welchen hydrophile Hautpflegewirkstoffe über einen langen Zeitraum stabil gelagert werden können und welche ein gutes Transportmittel für diese Wirkstoffe darstellen.

Es war überraschend und für den Fachmann nicht vorauszusehen, daß kosmetische und dermatologische Formulierungen mit mindestens einem hydrophilen Wirkstoff gewählt aus der Gruppe
- Biotin
- Carnitin und Derivate
- Creatin und Derivate
- Folsäure
- Pyridoxin
- Niacinamid
- Polyphenole (insbesondere Flavonoide, ganz besonders alpha-Glucosylrutin)
- Ascorbinsäure und Derivate
- Hamamelis
- Aloe Vera
- Panthenol
- Aminosäuren
- Wasserlösliche Antioxidantien,
mit mindestens einem Dialkylnaphthalat, welches sich durch die Strukturformel 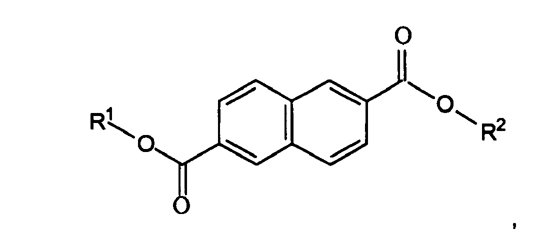 auszeichnet, worin R¹ und R² unabhängig voneinander gewählt werden aus der Gruppe der verzweigten und unverzweigten Alkylgruppen mit 6 bis 24 Kohlenstoffatomen, dadurch gekennzeichnet, daß sie mindestens eine UV-Filtersubstanz, gewählt aus der Gruppe Triazine, Benzotriazole, bei Raumtemperatur flüssigen UV-Filter und organische und/oder anorganische Pigmente, enthalten den Nachteilen des Standes der Technik abhelfen.

Die Zubereitungen im Sinne der vorliegenden Erfindung können bevorzugt neben einer oder mehrerer Ölphasen zusätzlich eine oder mehrere Wasserphasen enthalten und beispielsweise in Form von W/O-, O/W-, W/O/W- oder O/W/O-Emulsionen vorliegen. Solche Formulierungen können vorzugsweise auch eine Mikroemulsion, eine PIT-Emulsion, eine Feststoff-Emulsionen (d. h. eine Emulsion, welche durch Feststoffe stabilisiert ist, z. B. eine Pickering-Emulsion), eine sprühbare Emulsion oder eine Hydrodispersion sein.

Die erfindungsgemäßen Zubereitungen stellen in jeglicher Hinsicht überaus befriedigende Präparate dar, welche nicht auf eine eingeschränkte Rohstoffauswahl begrenzt sind. Dementsprechend eignen sie sich ganz besonders, um als Grundlage für Zubereitungsformen mit vielfältigen Anwendungszwecken zu dienen. Die erfindungsgemäßen Zubereitungen zeigen sehr gute sensorische und kosmetische Eigenschaften, wie beispielsweise die Verteilbarkeit auf der Haut oder das Einzugsvermögen in die Haut, und zeichen sich ferner durch eine sehr gute Wirkstoffeffektivität bei gleichzeitig hervorragenden Hautpflegedaten aus.

Es war für den Fachmann nicht vorauszusehen gewesen, daß die Verwendung von Alkylnaphthalaten hydrophile Wirkstoffe besser gegen den Zerfall stabilisieren würde. Ferner läßt sich durch die erfindungsgemäße Verwendung überraschend die Stabilität von hydrophilen Wirkstoffen in wasserhaltigen kosmetischen oder dermatologischen Formulierungen gegenüber dem Stand der Technik erheblich steigern.

Gegenstand der Erfindung ist daher auch die
Verwendung von mindestens einem Dialkylnaphthalat, welches sich durch die Strukturformel 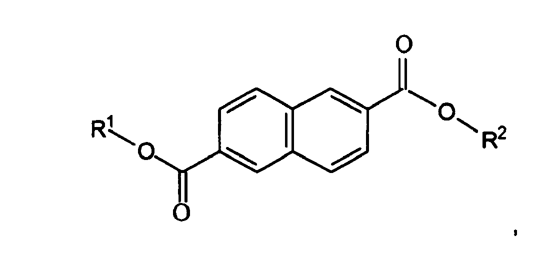 auszeichnet, worin R¹ und R² unabhängig voneinander gewählt werden aus der Gruppe der verzweigten und unverzweigten Alkylgruppen mit 6 bis 24 Kohlenstoffatomen, zur Verbesserung der Wirksamkeit und zur Erhöhung der Stabilität von hydrophilen Wirkstoffen in kosmetischen oder dermatologischen Zubereitungen.

Vorteilhafte hydrophile Wirkstoffe, welche (einzeln oder in beliebigen Kombinationen miteinander) durch die erfindungsgemäße Verwendung in hervorragender Weise stabilisiert werden, sind die im folgenden aufgelisteten:
- Biotin
- Carnitin und Derivate
- Creatin und Derivate
- Folsäure
- Pyridoxin
- Niacinamid
- Polyphenole (insbesondere Flavonoide, ganz besonders alpha-Glucosylrutin)
- Ascorbinsäure und Derivate
- Hamamelis
- Aloe Vera
- Panthenol
- Aminosäuren

Besonders vorteilhafte hydrophile Wirkstoffe im Sinne der vorliegenden Erfindung sind ferner wasserlösliche Antioxidantien, wie beispielsweise Vitamine.

Die Menge an hydrophilen Wirkstoffen (eine oder mehrere Verbindungen) in den Zubereitungen beträgt vorzugsweise 0,0001 bis 10 Gew.-%, besonders bevorzugt 0,001 bis 5 Gew.-%, bezogen auf das Gesamtgewicht der Zubereitung.

Vorteilhaft im Sinne der vorliegenden Erfindung sind Dialkylnaphthalate, für die R¹ und/oder R² verzweigte Alkylgruppen mit 6 bis 10 Kohlenstoffatomen darstellen. Ganz besonders bevorzugt im Sinne der vorliegenden Erfindung ist Diethylhexylnaphthalat, welches beispielsweise unter der Handelsbezeichnung Hallbrite TQ^{™} von CP Hall oder Corapan TQ^{™} von H&R erhältlich ist.

Erfindungsgemäß vorteilhaft enthalten kosmetische oder dermatologische Zubereitungen 0,001 bis 30 Gew.-%, vorzugsweise 0,01 bis 20 Gew.-%, ganz besonders bevorzugt 1 bis 15 Gew.-% eines oder mehrerer Dialkylnaphthalate.

Die erfindungsgemäßen kosmetischen oder dermatologischen Lichtschutzformulierungen können wie üblich zusammengesetzt sein und dem kosmetischen oder dermatologischen Lichtschutz, ferner zur Behandlung, Pflege und Reinigung der Haut und/oder der Haare und als Schminkprodukt in der dekorativen Kosmetik dienen.

Entsprechend ihrem Aufbau können kosmetische oder topische dermatologische Zusammensetzungen im Sinne der vorliegenden Erfindung, beispielsweise verwendet werden als Hautschutzcrème, Reinigungsmilch, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Zur Anwendung werden die kosmetischen und dermatologischen Zubereitungen in der für Kosmetika üblichen Weise auf die Haut und/oder die Haare in ausreichender Menge aufgebracht.

Die kosmetischen und dermatologischen Zubereitungen gemäß der Erfindung können kosmetische Hilfsstoffe enthalten, wie sie üblicherweise in solchen Zubereitungen verwendet werden, z. B. Konservierungsmittel, Konservierungshelfer, Bakterizide, Parfüme, Substanzen zum Verhindern des Schäumens, Farbstoffe, Pigmente, die eine färbende Wirkung haben, Verdickungsmittel, anfeuchtende und/oder feuchhaltende Substanzen, Füllstoffe, die das Hautgefühl verbessern, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Vorteilhafte Konservierungsmittel im Sinne der vorliegenden Erfindung sind beispielsweise Formaldehydabspalter (wie z. B. DMDM Hydantoin, welches beispielsweise unter der Handelsbezeichnung Glydant^{™} von der Fa. Lonza erhältlich ist), lodopropylbutylcarbamate (z. B. die unter den Handelsbezeichnungen Glycacil-L, Glycacil-S von der Fa. Lonza erhältlichen und/oder Dekaben LMB von Jan Dekker), Parabene (d. h. p-Hydroxybenzoesäurealkylester, wie Methyl-, Ethyl-, Propyl- und/oder Butylparaben), Phenoxyethanol, Ethanol, Benzoesäure und dergleichen mehr. Üblicherweise umfaßt das Konservierungssystem erfindungsgemäß ferner vorteilhaft auch Konservierungshelfer, wie beispielsweise Octoxyglycerin, Glycine Soja etc.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Es ist insbesondere vorteilhaft, wenn die kosmetischen Zubereitungen gemäß der vorliegenden Erfindung weitere kosmetische oder dermatologische Wirkstoffe enthalten, wobei bevorzugte Wirkstoffe Antioxidantien sind, welche die Haut vor oxidativer Beanspruchung schützen können.

Vorteilhafte weitere Wirkstoffe sind natürliche Wirkstoffe und/oder deren Derivate, wie z. B. Ubichinone, Retinoide, Carotinoide, Kreatin, Taurin und/oder β-Alanin.

Erfindungsgemäße Rezepturen, welche z. B. bekannte Antifaltenwirkstoffe wie Flavonglycoside (insbesondere α-Glycosylrutin), Coenzym Q10, Vitamin E und/oder Derivate und dergleichen enthalten, eignen sich insbesondere vorteilhaft zur Prophylaxe und Behandlung kosmetischer oder dermatologischer Hautveränderungen, wie sie z. B. bei der Hautalterung auftreten (wie beispielsweise Trockenheit, Rauhigkeit und Ausbildung von Trockenheitsfältchen, Juckreiz, verminderte Rückfettung (z. B. nach dem Waschen), sichtbare Gefäßerweiterungen (Teleangiektasien, Cuperosis), Schlaffheit und Ausbildung von Falten und Fältchen, lokale Hyper-, Hypo- und Fehlpigmentierungen (z. B. Altersflecken), vergrößerte Anfälligkeit gegenüber mechanischem Stress (z. B. Rissigkeit) und dergleichen). Weiterhin vorteilhaft eignen sie sich gegen das Erscheinungsbild der trockenen bzw. rauhen Haut.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Ferner vorteilhaft sind Copolymere aus C₁₀₋₃₀-Alkylacrylaten und einem oder mehreren Monomeren der Acrylsäure, der Methacrylsäure oder deren Ester, die kreuzvernetzt sind mit einem Allylether der Saccharose oder einem Allylether des Pentaerythrit.

Vorteilhaft sind Verbindungen, die die INCI-Bezeichnung "Acrylates/C₁₀₋₃₀ Alkyl Acrylate Crosspolymer" tragen. Insbesondere vorteilhaft sind die unter den Handelsbezeichnungen Pemulen TR1 und Pemulen TR2 bei der B. F. Goodrich Company erhältlichen.

Vorteilhaft sind Verbindungen, die die INCI-Bezeichnung Ammoniumacryloyldimethyltaurate/Vinylpyrrolidoncopolymere tragen.

Erfindungsgemäß vorteilhaft weisen das oder die Ammoniumacryloyldimethyltaurate/Vinylpyrrolidoncopolymere die Summenformel [C₇H₁₆N₂SO₄]ₙ[C₆H₉NO]ₘ auf, einer statistischen Struktur wie folgt entsprechend

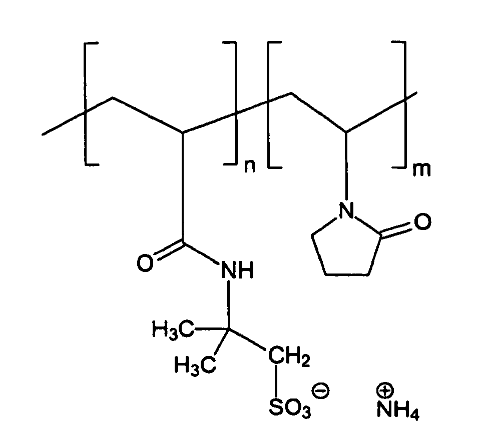

Bevorzugte Spezies im Sinne der vorliegenden Erfindung sind in den Chemical Abstracts unter den Registraturnummern 58374-69-9, 13162-05-5 und 88-12-0 abgelegt und erhältlich unter der Handelsbezeichnung Aristoflex® AVC der Gesellschaft Clariant GmbH.

Vorteilhaft sind ferner Copolymere/Crosspolymere umfassend Acryloyldimethyl Taurate, wie beispielsweise Simugel ® EG oder Simugel ® EG von der Gesellschaft Seppic S.A.

Auch Moisturizer können bevorzugt verwendet werden.

Als Moisturizer werden Stoffe oder Stoffgemische bezeichnet, welche kosmetischen oder dermatologischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Moisturizer im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist.

Die erfindungsgemäßen kosmetischen oder dermatologischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile^{®} (CAS-Nr. 7631-86-9).

Die Ölphase der erfindungsgemäßen Formulierungen wird vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol CC* bei der Fa. Cognis erhältliche.

Es ist ferner bevorzugt, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der erfindungsgemäßen Formulierungen einen Gehalt an C₁₂₋₁₅-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hallbrite BHB* bei der Fa. CP Hall erhältliche), Hexadecylbenzoat und Butyloctylbenzoat und Gemische davon (*Hallstar AB*)*.*

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene und Isohexadecan. Unter den Polyolefinen sind Polydecene die bevorzugten Substanzen.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Silikonöle sind hochmolekulare synthetische polymere Verbindungen, in denen Silicium-Atome über Sauerstoff-Atome ketten- und/oder netzartig verknüpft und die restlichen Valenzen des Siliciums durch Kohlenwasserstoff-Reste (meist Methyl-, seltener Ethyl-, Propyl-, PhenylGruppen u. a.) abgesättigt sind. Systematisch werden die Silikonöle als Polyorganosiloxane bezeichnet. Die methylsubstituierten Polyorganosiloxane, welche die mengenmäßig bedeutendsten Verbindungen dieser Gruppe darstellen und sich durch die folgende Strukturformel auszeichnen 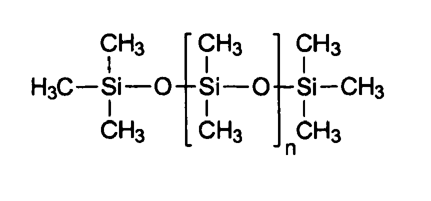 werden auch als Polydimethylsiloxan bzw. Dimethicon (INCI) bezeichnet. Dimethicone gibt es in verschiedenen Kettenlängen bzw. mit verschiedenen Molekulargewichten.

Besonders vorteilhafte Polyorganosiloxane im Sinne der vorliegenden Erfindung sind beispielsweise Dimethylpolysiloxane [Poly(dimethylsiloxan)], welche beispielsweise unter den Handelsbezeichnungen Abil 10 bis 10 000 bei Th. Goldschmidt erhältlich sind. Ferner vorteilhaft sind Phenylmethylpolysiloxane (INCI: Phenyl Dimethicone, Phenyl Trimethicone), cyclische Silikone (Octamethylcyclotetrasiloxan bzw. Decamethylcyclopentasiloxan), welche nach INCI auch als Cyclomethicone bezeichnet werden, aminomodifizierte Silikone (INCI: Amodimethicone) und Silikonwachse, z. B. Polysiloxan-Polyalkylen-Copolymere (INCI: Stearyl Dimethicone und Cetyl Dimethicone) und Dialkoxydimethylpolysiloxane (Stearoxy Dimethicone und Behenoxy Stearyl Dimethicone), welche als verschiedene Abil-Wax-Typen bei Th. Goldschmidt erhältlich sind. Aber auch andere Silikonöle sind vorteilhaft im Sinne der vorliegenden Erfindung zu verwenden, beispielsweise Cetyldimethicon, Hexamethylcyclotrisiloxan, Polydimethylsiloxan, Poly(methylphenylsiloxan).

Es ist auch vorteilhaft im Sinne der vorliegenden Erfindung, kosmetische und dermatologische Zubereitungen zu erstellen, deren hauptsächlicher Zweck nicht der Schutz vor Sonnenlicht ist, die aber dennoch einen Gehalt an weiteren UV-Schutzsubstanzen enthalten. So werden z. B. in Tagescrèmes oder Makeup-Produkten gewöhnlich UV-A- bzw. UV-B-Filtersubstanzen eingearbeitet. Auch stellen UV-Schutzsubstanzen, ebenso wie Antioxidantien und, gewünschtenfalls, Konservierungsstoffe, einen wirksamen Schutz der Zubereitungen selbst gegen Verderb dar. Günstig sind ferner kosmetische und dermatologische Zubereitungen, die in der Form eines Sonnenschutzmittels vorliegen.

Dementsprechend enthalten die Zubereitungen im Sinne der vorliegenden Erfindung vorzugsweise mindestens eine weitere UV-A-, UV-B- und/oder Breitbandfiltersubstanz. Die Formulierungen können, obgleich nicht notwendig, gegebenenfalls auch ein oder mehrere organische und/oder anorganische Pigmente als UV-Filtersubstanzen enthalten, welche in der Wasser- und/oder der Ölphase vorliegen können.

Die erfindungsgemäßen Zubereitungen können ferner vorteilhaft auch in Form von sogenannten ölfreien kosmetischen oder dermatologischen Emulsionen vorliegen, welche eine Wasserphase und mindestens eine bei Raumtemperatur flüssige UV-Filtersubstanz und/oder ein oder mehrere Silikonderivate als weitere Phase enthalten. Ölfreie Formulierungen im Sinne der vorliegenden Erfindung können vorteilhaft auch weitere lipophile Komponenten - wie beispielsweise lipophile Wirkstoffe - enthalten.

Besonders vorteilhafte bei Raumtemperatur flüssige UV-Filtersubstanzen im Sinne der vorliegenden Erfindung sind Homomenthylsalicylat (INCI: Homosalate), 2-Ethylhexyl-2-cyano-3,3-diphenylacrylat (INCI: Octocrylene), 2-Ethylhexyl-2-hydroxybenzoat (2-Ethylhexylsalicylat, Octylsalicylat, INCI: Octyl Salicylate) und Ester der Zimtsäure, vorzugsweise 4-Methoxyzimtsäure(2-ethylhexyl)ester (2-Ethylhexyl-4-methoxycinnamat, INCI: Octyl Methoxycinnamate) und 4-Methoxyzimtsäureisopentylester (Isopentyl-4-methoxycinnamat, INCI: Isoamyl p-Methoxycinnamate).

Bevorzugte anorganische Pigmente sind Metalloxide und/oder andere in Wasser schwerlösliche oder unlösliche Metallverbindungen, insbesondere Oxide des Titans (TiO₂), Zinks (ZnO), Eisens (z. B. Fe₂O₃), Zirkoniums (ZrO₂), Siliciums (SiO₂), Mangans (z. B. MnO), Aluminiums (Al₂O₃), Cers (z. B. Ce₂O₃), Mischoxide der entsprechenden Metalle sowie Abmischungen aus solchen Oxiden sowie das Sulfat des Bariums (BaSO₄).

Die Pigmente können vorteilhaft im Sinne der vorliegenden Erfindung auch in Form kommerziell erhältlicher öliger oder wäßriger Vordispersionen zur Anwendung kommen. Diesen Vordispersionen können vorteilhaft Dispergierhilfsmittel und/oder Solubilisationsvermittler zugesetzt sein.

Die Pigmente können erfindungsgemäß vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, daß die Pigmente nach an sich bekannten Verfahren mit einer dünnen hydrophilen und/oder hydrophoben anorganischen und/oder organischen Schicht versehen werden. Die verschiedenen Oberflächenbeschichtungen können im Sinne der vorliegenden Erfindung auch Wasser enthalten.

Anorganische Oberflächenbeschichtungen im Sinne der vorliegenden Erfindung können bestehen aus Aluminiumoxid (Al₂O₃), Aluminiumhydroxid Al(OH)₃, bzw. Aluminiumoxidhydrat (auch: Alumina, CAS-Nr.: 1333-84-2), Natriumhexametaphosphat (NaPO₃)₆, Natriummetaphosphat (NaPO₃)ₙ, Siliciumdioxid (SiO₂) (auch: Silica, CAS-Nr.: 7631-86-9), oder Eisenoxid (Fe₂O₃). Diese anorganischen Oberflächenbeschichtungen können allein, in Kombination und/oder in Kombination mit organischen Beschichtungsmaterialien vorkommen.

Organische Oberflächenbeschichtungen im Sinne der vorliegenden Erfindung können bestehen aus pflanzlichem oder tierischem Aluminiumstearat, pflanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel) oder Alginsäure. Diese organischen Oberflächenbeschichtungen können allein, in Kombination und/oder in Kombination mit anorganischen Beschichtungsmaterialien vorkommen.

Erfindungsgemäß geeignete Zinkoxidpartikel und Vordispersionen von Zinkoxidpartikeln sind unter folgenden Handelsbezeichnungen bei den aufgeführten Firmen erhältlich:

| **Handelsname** | **Coating** | **Hersteller** |
|---|---|---|
| Z- Cote HP1 | 2% Dimethicone | BASF |
| Z- Cote | / | BASF |
| ZnO NDM | 5% Dimethicone | H&R |
| ZnO Neutral | / | H&R |
| ZnO MZ303 M | 3% Methicone | Tayca Corp. |

Geeignete Titandioxidpartikel und Vordispersionen von Titandioxidpartikeln sind unter folgenden Handelsbezeichnungen bei den aufgeführten Firmen erhältlich:

| **Handelsname** | **Coating** | **Hersteller** |
|---|---|---|
| MT-100TV | Aluminiumhydroxid / Stearinsäure | Tayca Corporation |
| MT-100Z | Aluminiumhydroxid / Stearinsäure | Tayca Corporation |
| Eusolex T-2000 | Alumina / Simethicone | Merck KgaA |
| Titandioxid T805 (Uvinul TiO₂) | Octyltrimethylsilan | Degussa |
| Tioveil AQ 10PG | Alumina / Silica | Solaveil / Uniquema |

Weitere vorteilhafte Pigmente sind Latexpartikel. Erfindungsgemäß vorteilhafte Latexpartikel sind die in den folgenden Schriften beschriebenen: US 5,663,213 bzw. EP 0 761 201. Besonders vorteilhafte Latexpartikel sind solche, welche aus Wasser und Styrol/Acrylat-Copolymeren gebildet werden und z. B. unter der Handelsbezeichnung "Alliance SunSphere" bei der Fa. Rohm & Haas erhältlich sind.

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol^{®} 1789 und von Merck unter der Handelsbezeichnung Eusolex® 9020 verkauft wird.

Vorteilhafte weitere UV-Filtersubstanzen im Sinne der vorliegenden Erfindung sind sulfonierte, wasserlösliche UV-Filter, wie z. B.
- Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz mit der INCI-Bezeichnung Bisimidazylate (CAS-Nr.: 180898-37-7), welches beispielsweise unter der Handelsbezeichnung Neo Heliopan AP bei Haarmann & Reimer erhältlich ist;
- Salze der 2-Phenylbenzimidazol-5-sulfonsäure, wie ihr Natrium-, Kalium- oder ihr Triethanolammonium-Salz sowie die Sulfonsäure selbst mit der INCI Bezeichnung Phenylbenzimidazole Sulfonsäure (CAS.-Nr. 27503-81-7), welches beispielsweise unter der Handelsbezeichnung Eusolex 232 bei Merck oder unter Neo Heliopan Hydro bei Haarmann & Reimer erhältlich ist;
- 1,4-di(2-oxo-10-Sulfo-3-bornylidenmethyl)-Benzol (auch: 3,3'-(1,4-Phenylendimethylene)-bis-(7,7-dimethyl-2-oxo-bicyclo-[2.2.1]hept-1-ylmethan Sulfonsäure) und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bomylidenmethyl-10-sulfonsäure) bezeichnet wird. Benzol-1,4-di(2-oxo-3-bomylidenmethyl-10-sulfonsäure) hat die INCI-Bezeichnung Terephtalidene Dicampher Sulfonsäure (CAS.-Nr.: 90457-82-2) und ist beispielsweise unter dem Handelsnamen Mexoryl SX von der Fa. Chimex erhältlich;
- Sulfonsäure-Derivate des 3-Benzylidencamphers, wie z. B. 4-(2-Oxo-3-bornylidenmethyl)-benzolsulfonsäure, 2-Methyl-5-(2-oxo-3-bornylidenmethyl)sulfonsäure und deren Salze.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Triazinderivate, wie z. B.
- 2,4-Bis-{[4-(2-Ethyl-hexyloxy)-2-hydroxy]-phenyl}-6-(4-methoxyphenyl)-1,3,5-triazin (INCI: Methylene Bis-Benzotriazolmethylbutylphenol), welches unter der Handelsbezeichnung Tinosorb® S bei der CIBA-Chemikalien GmbH erhältlich ist;
- Dioctylbutylamidotriazon (INCI: Diethylhexylbutamidotriazone), welches unter der Handelsbezeichnung UVASORB HEB bei Sigma 3V erhältlich ist;
- 4,4',4"-(1,3,5-Triazin-2,4,6-triyltriimino)-tris-benzoësäure-tris(2-ethylhexylester), auch: 2,4,6-Tris-[anilino-(p-carbo-2'-ethyl-1'-hexyloxy)]-1,3,5-triazin (INCI: Octyl Triazorie), welches von der BASF Aktiengesellschaft unter der Warenbezeichnung UVINUL® T 150 vertrieben wird.

Ein vorteilhafter Breitbandfilter im Sinne der vorliegenden Erfindung ist auch das 2,2'-Methylen-bis-(6-(2H-benzotriazol-2-yl)-4-(1,1,3,3-tetramethylbutyl)-phenol) (INCI: Bisoctyltriazol), welches unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane.

Die weiteren UV-Filtersubstanzen können öllöslich sein. Vorteilhafte öllösliche Filtersubstanzen sind z. B.:
■ 3-Benzylidencampher-Derivate, vorzugsweise 3-(4-Methylbenzyliden)campher, 3-Benzylidencampher;
■ 4-Aminobenzoesäure-Derivate, vorzugsweise 4-(Dimethylamino)-benzoesäure(2-ethylhexyl)ester, 4-(Dimethylamino)benzoesäureamylester;
■ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon
■ sowie an Polymere gebundene UV-Filter.

Eine weiterere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul^{®} N 539 erhältlich ist.

Besonders vorteilhafte Zubereitungen im Sinne der vorliegenden Erfindung, die sich durch einen hohen bzw. sehr hohen UV-A-Schutz auszeichnen, enthalten neben der oder den erfindungsgemäßen Filtersubstanz(en) bevorzugt ferner weitere UV-A- und/oder Breitbandfilter, insbesondere Dibenzoylmethanderivate [beispielsweise das 4-(tert.-Butyl)-4'-methoxydibenzoylmethan] und/oder das 2,4-Bis-{[4-(2-Ethyl-hexyloxy)-2-hydroxy]-phenyl}-6-(4-methoxyphenyl)-1,3,5-triazin, jeweils einzeln oder in beliebigen Kombinationen miteinander.

Die Liste der genannten UV-Filter, die im Sinne der vorliegenden Erfindung eingesetzt werden können, soll selbstverständlich nicht limitierend sein.

Vorteilhaft enthalten die erfindungsgemäßen Zubereitungen die Substanzen, die UV-Strahlung im UV-A- und/oder UV-B-Bereich absorbieren, in einer Gesamtmenge von z. B. 0,1 Gew.-% bis 30 Gew.-%, vorzugsweise 0,5 bis 20 Gew.-%, insbesondere 1,0 bis 15,0 Gew.-%, jeweils bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen.

Ferner kann es gegebenenfalls von Vorteil sein, Filmbildner in die erfindungsgemäßen kosmetischen oder dermatologischen Zubereitungen einzuarbeiten, beispielsweise um die Wasserfestigkeit der Zubereitungen zu verbessern oder die UV-Schutzleistung zu erhöhen (UV-A- und/oder UV-B-Boosting). Geeignet sind sowohl wasserlösliche bzw. dispergierbare als auch fettlösliche Filmbildner, jeweils einzeln oder in Kombination miteinander.

Vorteilhafte wasserlöslich bzw. dispergierbare Filmbildner sind z. B. Polyurethane (z. B. die Avalure® -Typen von Goodrich), Dimethicone Copolyol Polyacrylate (Silsoft Surface® von der Witco Organo Silicones Group), PVPNA (VA = Vinylacetat) Copolymer (Luviscol VA 64 Powder der BASF) etc.

Vorteilhafte fettlösliche Filmbildner sind z. B., die Filmbildner aus der Gruppe der Polymere auf Basis von Polyvinylpyrrolidon (PVP)

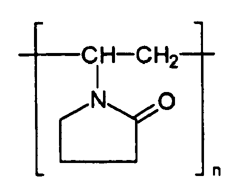

Besonders bevorzugt sind Copolymere des Polyvinylpyrrolidons, beispielsweise das PVP Hexadecen Copolymer und das PVP Eicosen Copolymer, welche unter den Handelsbezeichnungen Antaron V216 und Antaron V220 bei der GAF Chemicals Cooperation erhältlich sind, sowie das Tricontayl PVP und dergleichen mehr.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

### Beispiele:

### 1. O/W Sonnenschutz Emulsionen

| | **1** | **2** | **3** | **4** | **5** | **6** | **7** |
|---|---|---|---|---|---|---|---|
| Glycerinmonostearat SE | 0,50 | 1,00 | 3,00 | | | 1,50 | |
| Glyceryl Stearat Citrat | 2,00 | | | 1,00 | 2,00 | | 2,50 |
| Stearinsäure | | 3,00 | | 2,00 | | | |
| PEG-40 Stearat | 0,50 | | | | | 2,00 | |
| Cetyl Dimethicon Copolyol | | | | 0,75 | | 0,50 | |
| Cetyl Phosphat | | | | | 1,00 | | |
| Stearyl Alkohol | | | 3,00 | | | 2,00 | 0,50 |
| Cetyl Alkohol | 2,50 | 1,00 | | 1,50 | 0,50 | | 2,00 |
| Ethylhexyl Methoxycinnamat | | | | | 6,00 | | 8,00 |
| Aniso Triazin | | 1,50 | 2,50 | | 2,50 | | 2,50 |
| Butyl Methoxydibenzoylmethan | 1,00 | 3,00 | | | 2,80 | | 1.50 |
| Bisimidazylat | | 0.50 | | | | 1,70 | |
| Ethylhexyl Triazon | 4,00 | | 3,00 | | 4,00 | 2,00 | |
| Octocrylen | | 4,00 | | | | | 2,50 |
| Diethylhexyl Butamido Triazon | 1,00 | | | | 1,00 | | |
| Phenylbenzmidazol Sulfonsäure | 0,50 | | | | | | |
| Bioctyltriazol | 2,00 | | 0,50 | | 2,50 | | |
| Homosalat | | 2,00 | | | | | |
| Ethylhexylsalicylat | | | 3,00 | | | | 5,00 |
| Drometrizol Trisiloxan | | | 0,5 | | | 1,00 | |
| Terephthaliden Dicamphor Sulfonsäure | | 1,50 | | | 1,00 | 0,50 | |
| Diethylhexyl-2,6-naphthalat | 3,50 | 4,80 | 7,00 | 9,50 | 6,70 | 5,50 | 8,00 |
| Titandioxid MT-100Z | 1,00 | | | | 3,00 | 2,00 | |
| Zinkoxid NDM | | | 1,50 | | | 2,00 | 3,00 |
| C12-15 Alkyl Benzoat | | 2,50 | | | 4,00 | 7,00 | 5,00 |
| Dicaprylyl Ether | | | 3,50 | | 2,00 | | |
| Butylenglycol Dicaprylat/Dicaprat | 5,00 | | | 6,00 | | | |
| Dibutyl Adipat | | | 6,00 | | | 2,00 | 2,00 |
| Dimethicon | | 0,50 | 1,00 | | 2,00 | | |
| Cyclomethicon | 2,00 | | | 0,50 | | | 0,50 |
| Shea Butter | | 2,00 | | | | | 0,50 |
| PVP Hexadecen Copolymer | 0,50 | | | 0,50 | 1,00 | | 1,00 |
| Tricontanyl PVP | | 0,50 | 1,00 | | | | 1,00 |
| Glycerin | 3,00 | 7,50 | | 7,50 | 5,00 | | 2,50 |
| Xanthan Gummi | 0,15 | | 0,05 | | | | 0,30 |
| Sodium Carbomer | | 0,20 | 0,10 | 0,20 | | | |
| Vitamin E Acetat | 0,50 | | 0,25 | 0,50 | 0,75 | | 1,00 |
| Alpha-Glucosylrutin | 0,25 | 0,75 | | 0,20 | | | |
| Ascorbinsäure | | | | | 2,50 | | |
| Panthenol | | | 1,50 | | | 5,00 | |
| Aloe Vera | | | 1,50 | | | | 3,50 |
| DMDM Hydantoin | | 0,60 | 0,40 | 0,20 | | | |
| Konkaben LMB ® | | | | 0,18 | 0,20 | 0,10 | 0,15 |
| Methylparaben | 0,15 | | 0,25 | | 0,50 | | |
| Phenoxyethanol | 1,00 | 0,40 | | 0,40 | 0,50 | 0,40 | 0,60 |
| EDTA | | 0,20 | 0,35 | 0,50 | 0,02 | | 0,03 |
| Iminodibernsteinsäure | 0,35 | 0,10 | | | 0,10 | | |
| Ethanol | | 2,00 | 1,50 | | 3,00 | | 1,00 |
| Parfüm | 0,20 | 0,20 | | | | 0,20 | 0,20 |
| Wasser | ad 100 | ad 100 | ad 100 | ad 100 | ad 100 | ad 100 | ad 100 |

### 2. Hydrodispersionen

| | **1** | **2** | **3** | **4** | **5** |
|---|---|---|---|---|---|
| PEG-40 Stearat | 1,00 | | | 0,5 | |
| Cetyl Alkohol | | | 1,00 | | |
| Sodium Carbomer | | 0,20 | | 0,30 | |
| Acrylates/C10-30 Alkyl Acrylat Crosspolymer | 0,50 | | 0,40 | 0,10 | 0,10 |
| Xanthan Gummi | | 0,30 | 0,15 | | 0,50 |
| Ethylhexyl Methoxycinnamat | | | | 5,00 | 8,00 |
| Aniso Triazin | | | | 2,00 | 2,50 |
| Butyl Methoxydibenzoylmethan | | 0.50 | 2,00 | 3.00 | 2,50 |
| Bisimidazylat | | | 1,50 | | 3.00 |
| Ethylhexyl Triazon | | | 3,00 | 4,00 | |
| Octocrylen | | 4,00 | 3,90 | | 2,50 |
| Diethyhexyl Butamido Triazon | | | | 2,00 | |
| Phenylbenzmidazol Sulfonsäure | | | | 3,00 | |
| Drometrizol Trisiloxan | | | 1,00 | | 1,50 |
| Terephthaliden Dicamphor Sulfonsäure | | 0,50 | | | 1,00 |
| Diethylhexyl-2,6-naphthalat | 4,50 | 8,00 | 7,20 | 5,50 | 9,80 |
| Titandioxid MT-100TV | | | 2,00 | | 1,00 |
| Zinkoxid HP1 | | | | | 3,00 |
| C12-15 Alkyl Benzoat | 2,00 | 2,50 | | | |
| Butylenglycol Dicaprylat/Dicaprat | 4,00 | | 2,00 | 6,00 | |
| Dicaprylyl Carbonat | | 2,00 | 6,00 | | |
| Dimethicon | | 0,50 | 1,00 | | |
| Shea Butter | | 2,00 | | 3,50 | 1,50 |
| PVP Hexadecen Copolymer | 0,50 | | | 0,50 | 1,00 |
| Ethylhexylglycerin | | | 1,00 | | 0,50 |
| Glycerin | 3,00 | 7,50 | | 7,50 | 2,50 |
| Glycin Soja | | | 1,50 | | |
| Vitamin E | 0,50 | | 0,25 | | 1,00 |
| Alpha-Glucosylrutin | 1,00 | 0,25 | 0,75 | | |
| Ascorbyl-Palmitat | 0,25 | | | 0,65 | 0,40 |
| DMDM Hydantoin | | 0,60 | 0,40 | 0,20 | |
| Konkaben LMB ® | 0,20 | | | | 0,15 |
| Methylparaben | 0,50 | | 0,25 | 0,15 | |
| Phenoxyethanol | 0,50 | 0,40 | | 1,00 | 0,60 |
| Iminodibersteinsäure | 0,50 | | 0,35 | 1,00 | |
| Ethanol | 3,00 | 2,00 | 1,50 | | 1,00 |
| Parfüm | 0,20 | | 0,20 | | 0,20 |
| Wasser | ad 100 | ad 100 | ad 100 | ad 100 | ad 100 |

### 3. W/O Sonnenschutz Emulsionen

| | **1** | **2** | **3** | **4** | **5** |
|---|---|---|---|---|---|
| Cetyldimethicon Copolyol | | 2,50 | | 4,00 | |
| Polyglyceryl-2-dipolyhydroxystearat | 5,00 | | | | 7,00 |
| PEG-30-dipolyhydroxystearat | | | 5,00 | | |
| Laurylmethicon Copolyol | 3,00 | | 2,00 | | |
| Ethylhexyl Methoxycinnamat | | 8,00 | | 5,00 | 4,00 |
| Aniso Triazin | | 2,50 | 2,00 | 2,00 | 2,50 |
| Butyl Methoxydibenzoylmethan | | 3,00 | | 1,00 | 0,70 |
| Bisimidazylat | | | | 2,00 | 2,60 |
| Ethylhexyl Triazon | | | 3,00 | 4,00 | |
| 4-Methylbenzyliden Campher | | 2,00 | | 4,00 | 2,00 |
| Octocrylen | | 2,50 | 3,90 | | 2,50 |
| Diethyhexyl Butamido Triazon | | | | 2,00 | |
| Phenylbenzmidazol Sulfonsäure | | | | 3,00 | 2,00 |
| Bisoctyl Triazol | | | 2,00 | 0,50 | |
| Dimethicon Diethylbenzalmalonat | | | 1,50 | | 0,50 |
| Drometrizol Trisiloxan | | 1,00 | | | 1,50 |
| Terephthaliden Dicamphor Sulfonsäure | | | 1,00 | | 0,50 |
| Diethylhexyl-2,6-naphthalat | 7,50 | 5,50 | 8,50 | 4,00 | 12,00 |
| Titandioxid T805 | | 2,00 | 1,50 | | 3,00 |
| Z-Cote HP1 | | | | 8,00 | 2,00 |
| Mineralöl | 5,00 | | 10,0 | | 8,00 |
| C12-15 Alkyl Benzoat | | 5,00 | | 9,00 | |
| Dicaprylyl Ether | 10,00 | | | | 7,00 |
| Butylenglycol Dicaprylat/Dicaprat | | | 2,00 | 8,00 | 4,00 |
| Dicaprylyl Carbonat | 5,00 | | 6,00 | | |
| Dimethicon | | 4,00 | 1,00 | 5,00 | |
| Cyclomethicon | 2,00 | 25,00 | | | 2,00 |
| Shea Butter | 2,00 | | 3,00 | | |
| PVP Hexadecen Copolymer | 0,50 | | | 0,50 | 1,00 |
| Ethylhexylglycerin | | 0,30 | 1,00 | | 0,50 |
| Glycerin | 3,00 | 7,50 | | 7,50 | 2,50 |
| Glycin Soja | | 1,00 | 1,50 | | |
| MgSO₄ | 1,00 | 0,50 | | 0,50 | |
| Vitamin E Acetat | 0,50 | | 0,25 | | 1,00 |
| Alpha- Glucosylrutin | 0,50 | | | 1,00 | |
| Aloe Vera | 0,15 | 0,50 | 4,00 | 1,50 | |
| Biotin | | | | | 3,00 |
| DMDM Hydantoin | | 0,60 | 0,40 | 0,20 | |
| Methylparaben | 0,50 | | 0,25 | 0,15 | |
| Phenoxyethanol | 0,50 | 0,40 | | 1,00 | 0,60 |
| EDTA | 0,20 | 0,35 | | 0,15 | |
| Ethanol | 3,00 | | 6,00 | | 1,00 |
| Parfüm | 0,20 | | | 0,20 | 0,20 |
| Wasser | ad 100 | ad 100 | ad 100 | ad 100 | ad 100 |

### 4. Feststoffstabilisierte Emulsionen

| | | | | | |
|---|---|---|---|---|---|
| | **1** | **2** | **3** | **4** | **5** |
| Mineralöl | | | 16,0 | 16,0 | |
| Octyldodecanol | 9,0 | 9,0 | 5,0 | | |
| Caprylic/Capric Triglycerid | 9,0 | 9,0 | 6,0 | | |
| C12-15- Alkyl Benzoat | | | | 5,0 | 8,0 |
| Butylen Glycol Dicaprylat/Dicaprat | | 3,5 | | | 8,0 |
| Dicaprylyl Ether | 9,0 | | | 4,0 | |
| Dicaprylyl Carbonat | | 9,0 | | | |
| Hydroxyoctacosanyl Hydroxystearat | 2,0 | 2,0 | 2,0 | 2,0 | 1,5 |
| Disteardimonium Hectorit | 1,0 | | 0,5 | 0,5 | 0,25 |
| Cera Microcristallina + Paraffinum Liquidum | | | | | 5,0 |
| Hydroxypropyl Methylcellulose | | 0,15 | | | 0,05 |
| Dimethicon | | | 2,50 | | 3,0 |
| Butyl methoxydibenzoylmethan | | 0,50 | 3,50 | 1,50 | 0,50 |
| Ethylhexylmethoxycinnamat | | | | | 3,0 |
| Diethylhexyl Butamido Triazon | | 2,50 | | | 4,0 |
| Bisoctyltriazol | | | 1,00 | 2,0 | |
| Drometrizol Trisiloxan | | 0,50 | | 1,00 | |
| Terephthaliden Dicamphor Sulfonsäure | | 1,00 | 0,50 | | 1,50 |
| Bisimidazylat | | 2,00 | 3.10 | 1,50 | 0,50 |
| Eusolex® T-2000 | | 2,0 | 4,0 | 2,0 | 4,0 |
| Titandioxid T 805 | | | | | 3,0 |
| Zinkoxid HP1 | | | | 6,0 | |
| Silica Dimethyl Silylat | | | 1,0 | | |
| Bornitrid | 2,0 | | | | |
| Stärke/-Natriummetaphosphat-Polymer | | 0,5 | | | |
| Diethylhexyl-2,6-naphthalat | 5.00 | 7.00 | 8.50 | 3.00 | 4.50 |
| Tapioca Stärke | | | | 1,0 | |
| Biotin | 0,20 | | 1,50 | 0,50 | |
| Alpha-Glucosylrutin | 1,50 | 2,00 | | | 3,00 |
| Natrium Chlorid | 1,0 | 1,0 | 1,0 | 1,0 | 1,0 |
| Glycerin | 5,0 | 10,0 | 3,0 | 6,0 | 10,0 |
| Trinatrium EDTA | | 1,0 | | 1,0 | |
| Methylparaben | 0,21 | | | | 0,2 |
| Propylparaben | 0,07 | | | | |
| Phenoxyethanol | 0,5 | | 0,4 | 0,4 | 0,5 |
| Hexamidin Diisethionat | | | | | 0,08 |
| Diazolidinyl Harnstoff | | | 0,28 | 0,28 | |
| Alcohol | | | | 2,5 | |
| Parfüm | q.s. | q.s. | q.s. | q.s. | q.s. |
| Wasser | ad 100 | ad 100 | ad 100 | ad 100 | ad 100 |

### 5. PIT-Emulsionen

| | **1** | **2** | **3** | **4** | **5** | **6** | **7** | **8** |
|---|---|---|---|---|---|---|---|---|
| Glycerinmonostearat SE | 5,50 | 2,00 | 3,00 | 5,00 | | | 0,50 | 4,00 |
| Glyceryl Isostearat | | | | | 3,50 | 4,00 | 2,00 | |
| Isoceteth-20 | | 0,50 | | | 2,00 | | | |
| Ceteareth-12 | 1,00 | 5,00 | | 1,00 | | | | 3,50 |
| Ceteareth-20 | 4,50 | | | 2,00 | | 2,50 | 3,00 | |
| PEG-100 Stearat | | | 1,00 | | 1,00 | | | 0,50 |
| Cetyl Alkohol | 2,50 | 1,00 | | 1,50 | | 0,50 | 1,50 | |
| Cetyl Palmitat | | | | 0,50 | | 1,00 | | |
| Cetyl Dimethicon Copolyol | 0,50 | | | | 0,50 | | 1,00 | |
| Polyglyceryl-2 Dipolyhydroxystearat | | | | 0,75 | 0,25 | | | |
| Diethylhexyl-2,6-naphthalat | 7,0 | 3,5 | 1,0 | 6,0 | 0,5 | 4,0 | 5,0 | 4,5 |
| Aniso Triazin | | | 0,50 | 2,00 | | 3,00 | | |
| Butyl Methoxydibenzoylmethan | | | 1,00 | | 5,00 | | | |
| Bisimidazylat | | 2,00 | | | 1,00 | | | |
| Terephthaliden Dicampher Sulfonsäure | | | 0,50 | | | | 1,00 | |
| Drometrizol Trisiloxan | | | 2,00 | | | 3,00 | | 1,00 |
| Ethylhexyl Methoxycinnamat | | | | 4,50 | 5,00 | 8,00 | | |
| Ethylhexyl Salicylat | | | | | 3,50 | 4,00 | | |
| Dioctyl Butamidotriazon | | | | 3,00 | 2,00 | 2,00 | | 1,50 |
| Ethylhexyl Triazon | | | 2,00 | 4,00 | | | 1,50 | 3,00 |
| Dimethicon Diethylbenzalmalonat | | 4,50 | | | 3,50 | | | |
| Octocrylen | | | 5,00 | | 8,00 | | | 7,50 |
| Phenylbenzmidazol Sulfonsäure | | 5,00 | | 3,00 | 1,00 | | | |
| C12-15 Alkyl Benzoat | 3,50 | | | | 6,50 | 4,00 | | |
| Cocoglyceride | | 3,00 | | 3,00 | | 2,50 | | 3,50 |
| Dicaprylyl Ether | 4,00 | | | | | | | |
| Butylenglycol Dicaprylat/Dicaprat | | 4,00 | | 3,00 | | | | |
| Dicaprylyl Carbonat | | | | 0,50 | | | | 6,00 |
| Dibutyl Adipate | | | 2,50 | | | 3,00 | | 1,00 |
| Cyclomethicon | | 3,00 | | | | | | 4,00 |
| PVP Hexadecen Copolymer | | | | 1,00 | 1,50 | | | |
| Glycerin | 10,0 | 5,00 | | 7,50 | | | | |
| Tocopherol | 1,00 | | | 0,75 | 0,50 | | 1,00 | 0,25 |
| alpha-Glucosylrutin | 0,45 | | 0,50 | | 1,00 | 0,25 | | 0,15 |
| Hammamelis-Extrakt | | 5,00 | | 10,00 | | 4,50 | | |
| Aloe Vera | | | | | | | 5,00 | 2,50 |
| Shea Butter | | 2,00 | | | | | | 0,50 |
| lodopropyl Butylcarbamt | 0,12 | | | | 0,20 | 0,15 | | |
| DMDM Hydantoin | | | | 0,10 | | | | |
| Methylparaben | | 0,50 | 0,25 | | 0,45 | | | |
| Phenoxyethanol | 0,50 | 0,40 | | 1,00 | | | | 1,00 |
| Octoxyglycerin | | 0,30 | | | 1,00 | | | |
| Ethanol | | | | 2,00 | | | 7,50 | 4,00 |
| Trisodium EDTA | | 0,40 | | 0,15 | | 0,20 | | 0,50 |
| Parfüm | 0,20 | | 0,20 | 0,20 | 0,45 | | | 0,20 |
| Wasser | ad. 100 | ad. 100 | ad. 100 | ad. 100 | ad. 100 | ad. 100 | ad. 100 | ad. 100 |

## Patentansprüche

1. Kosmetische Formulierung, welche mindestens einen hydrophilen Wirkstoff, gewählt aus der Gruppe:
- Biotin
- Carnitin und Derivate
- Creatin und Derivate
- Folsäure
- Pyridoxin
- Niacinamid
- Polyphenole (insbesondere Flavonoide, ganz besonders alpha-Glucosylrutin)
- Ascorbinsäure und Derivate
- Hamamelis
- Aloe Vera
- Panthenol
- Aminosäuren
- wasserlösliche Antioxidantien
und mindestens ein Dialkylnaphthalat, welches sich durch die Strukturformel 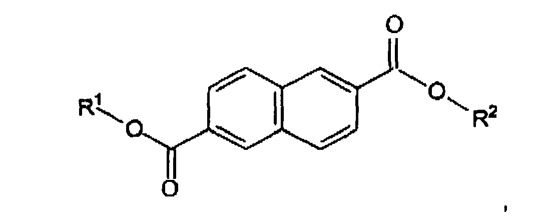 auszeichnet, worin R¹ und R² unabhängig voneinander gewählt werden aus der Gruppe der verzweigten und unverzweigten Alkylgruppen mit 6 bis 24 Kohlenstoffatomen, enthält, **dadurch gekennzeichnet, daß** sie mindestens eine UV-Filtersubstanz, gewählt aus der Gruppe Triazine, Benzotriazole, bei Raumtemperatur flüssigen UV-Filter und organische und/oder anorganische Pigmente, enthält.

2. Formulierung nach Anspruch 1, **dadurch gekennzeichnet, daß** der Gehalt an einem oder mehreren Dialkylnaphthalaten aus dem Bereich von 0,001 bis 30 Gew.-%, vorteilhaft 0,01 bis 20 Gew.-%, besonders bevorzugt 1 bis 15 Gew.-% gewählt wird, jeweils bezogen auf das Gesamtgewicht der Zubereitung.

3. Formulierung nach einem der vorangegangenen Ansprüche, **dadurch gekennzeichnet, daß** sie mindestens einen fettlöslichen Wirkstoff, insbesondere Vitamin E oder ein Vitamin E-Derivat enthält

4. Formulierung nach einem der vorhergehenden Ansprüche, **dadurch gekennzeichnet, dass** sie mindestens eine UV-A-Filtersubstanz und/oder einen Breitbandfilter, gewählt aus der Gruppe Dibenzoylmethanderivate [insbesondere das 4-(tert.-Butyl)-4'-methoxydibenzoyimethan] und 2,4-Bis-{[4-(2-Ethyl-hexyloxy)-2-hydroxy]-phenyl}-6-(4-methoxyphenyl)-1,3,5-triazin, Phenylen-1,4-bis-(2-benzimidazyl)-3,3'-5,5'-tetrasulfonsäure-bis-natriumsalz, enthält, wobei die weiteren Filtersubstanzen jeweils einzeln oder in beliebigen Kombinationen miteinander vorliegen können.

5. Kosmetische Verwendung von Formulierungen nach einem der vorhergehenden Ansprüche zur Hautbefeuchtung.

6. Kosmetische Verwendung von Formulierungen nach einem der Ansprüche 1 bis 4 zum Schutz vor lichtbedingter Hautalterung.

## Claims

1. Cosmetic formulation comprising at least one hydrophilic active ingredient chosen from the group consisting of
- biotin
- carnitine and derivatives
- creatine and derivatives
- folic acid
- pyridoxine
- niacinamide
- polyphenols (in particular flavonoids, very particularly alpha-glucosylrutin)
- ascorbic acid and derivatives
- Hamamelis
- Aloe Vera
- panthenol
- amino acids
- water-soluble antioxidants,
and
at least one dialkyl naphthalate which is **characterized by** the structural formula 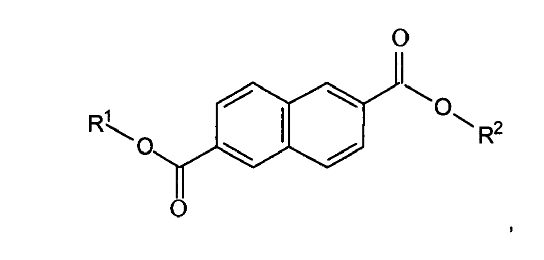 in which R¹ and R², independently of one another, are chosen from the group of branched and unbranched alkyl groups having 6 to 24 carbon atoms, **characterized in that** it comprises at least one UV filter substance chosen from the group consisting of triazines, benzotriazoles, UV filters which are liquid at room temperature, and organic and/or inorganic pigments.

2. Formulation according to Claim 1, **characterized in that** the content of one or more dialkyl naphthalates is chosen from the range from 0.001 to 30% by weight, advantageously 0.01 to 20% by weight, particularly preferably 1 to 15% by weight, in each case based on the total weight of the preparation.

3. Formulation according to any of the preceding claims, **characterized in that** it comprises at least one fat-soluble active ingredient, in particular vitamin E or a vitamin E derivative.

4. Formulation according to any of the preceding claims, **characterized in that** it comprises at least one UV-A filter substance and/or one broadband filter chosen from the group of dibenzoylmethane derivatives [in particular 4-(tert-butyl)-4'-methoxydibenzoylmethane] and 2,4-bis{[4-(2-ethylhexyloxy)-2-hydroxy]phenyl}-6-(4-methoxyphenyl)-1,3,5-triazine, phenylene-1,4-bis(2-benzimidazyl)-3,3'-5,5'-tetrasulfonic acid bis-sodium salt, where the further filter substances may in each case be present individually or in any combinations with one another.

5. Cosmetic use of formulations according to any of the preceding claims for moisturizing the skin.

6. Cosmetic use of formulations according to any of Claims 1 to 4 for protection against photoinduced skin ageing.

## Revendications

1. Formulation cosmétique qui contient au moins une substance active hydrophile, choisie dans le groupe formé par :
- la biotine
- la carnitine et ses dérivés
- la créatine et ses dérivés
- l'acide folique
- la pyridoxine
- le niacinamide
- les polyphénols (en particulier les flavonoïdes, tout particulièrement l'alpha-glucosylrutine)
- l'acide ascorbique et ses dérivés,
- l'hamamélis
- l'Aloe Vera
- le panthénol
- les acides aminés
- les antioxydants solubles dans l'eau
et au moins un naphtalate de dialkyle qui se distingue par la formule développée 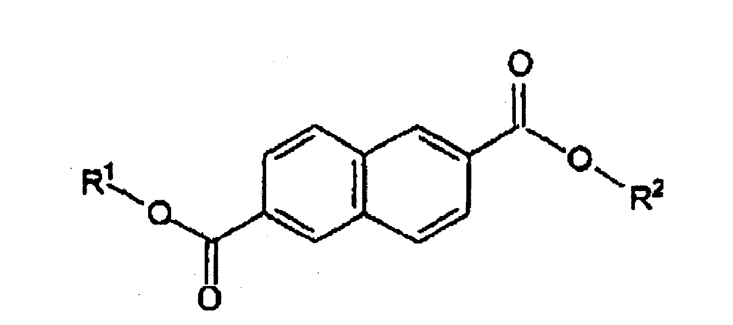 où R¹ et R² sont choisis indépendamment l'un de l'autre dans l'ensemble des groupes alkyle ramifiés et non ramifiés comprenant 6 à 24 atomes de carbone, **caractérisée en ce qu'**elle contient au moins une substance filtre des UV, choisie dans le groupe des triazines, des benzotriazoles, des filtres des UV liquides à température ambiante et des pigments organiques et/ou inorganiques.

2. Formulation selon la revendication 1, **caractérisée en ce que** la teneur en un ou plusieurs naphtalates de dialkyle est choisie dans la plage de 0,001 à 30% en poids, avantageusement de 0,01 à 20% en poids, de manière particulièrement préférée de 1 à 15% en poids, à chaque fois par rapport au poids total de la préparation.

3. Formulation selon l'une des revendications précédentes, **caractérisée en ce qu'**elle contient au moins une substance active soluble dans les graisses, en particulier la vitamine E ou un dérivé de vitamine E.

4. Formulation selon l'une quelconque des revendications précédentes, **caractérisée en ce qu'**elle contient au moins une substance filtre des UV-A et/ou un filtre à large bande, choisi(e)(s) dans le groupe formé par les dérivés de dibenzoylméthane [en particulier le 4-(tert-butyl)-4'-méthoxydibenzoylméthane] et la 2,4-bis-{[4-(2-éthylhexyloxy)-2-hydroxy]-phényl}-6-(4-méthoxyphényl)-1,3,5-triazine, le sel disodique de l'acide phénylène-1,4-bis-(2-benzimidazyl)-3,3'-5,5'-tétrasulfonique, les autres substances filtre pouvant à chaque fois être présentes seules ou dans des combinaisons quelconques les unes avec les autres.

5. Utilisation cosmétique de formulations selon l'une quelconque des revendications précédentes pour l'hydratation de la peau.

6. Utilisation cosmétique de formulations selon l'une quelconque des revendications 1 à 4 pour la protection contre le vieillissement de la peau provoqué par la lumière.
